(19) Europäisches Patentamt
European Patent Office
Office européen des brevets

(11) Veröffentlichungsnummer: 0 133 329
B1

(12) # EUROPÄISCHE PATENTSCHRIFT

(45) Veröffentlichungstag der Patentschrifft: 28.02.90

(51) Int. Cl.⁵: C 12 Q 1/40, C 12 Q 1/34

(21) Anmeldenummer: 84200866.6

(22) Anmeldetag: 15.06.84

(54) **Verfahren und Reagenz zur Bestimmung von alpha-Amylase.**

(30) Priorität: 28.06.83 DE 3323245

(43) Veröffentlichungstag der Anmeldung: 20.02.85 Patentblatt 85/08

(45) Bekanntmachung des Hinweises auf die Patenterteilung: 28.02.90 Patentblatt 90/09

(84) Bennante Vertragsstaaten: BE CH DE FR GB IT LI NL SE

(56) Entgegenhaltungen:
US-A-4 102 747
US-A-4 233 403

PATENT ABSTRACTS OF JAPAN, Band 5, Nr. 94 (C-59) 766, 19. Juni 1981; & JP-A-56 35 998 (TOYO BOSEKI K.K.) 08-04-1981

PATENT ABSTRACTS OF JAPAN, Band 6, Nr. 191 (C-127) 1069, 30. September 1982; & JP-A-57 102 198 (TOYO BOSEKI K.K.) 25-06-1982

CLINICAL CHEMISTRY, Band 28, Nr. 11, November 1982, Seiten 2201-2205, Washington, US; E.-O. HÄGELE et al.: "Mechanism of action of human pancreatic and salivary alpha-amylase on alpha-4-nitrophenyl maltoheptaoside substrate"

(73) Patentinhaber: MERCK PATENT GESELLSCHAFT MIT BESCHRÄNKTER HAFTUNG
Frankfurter Strasse 250 Postfach 4119
D-6100 Darmstadt (DE)

(72) Erfinder: Henkel, Eberhard, Dr.
Finkenhof 4a
D-3000 Hannover 61 (DE)
Erfinder: Dolabdjian, Barkew, Dr.
Im Lerchelsböhl 3
D-6080 Gross-Gerau (DE)
Erfinder: Helger, Roland, Dr.
Ludwigshöhstrasse 85
D-6100 Darmstadt (DE)
Erfinder: Klink, Rainer, Dr.
Heidelberger Landstrasse 16
D-6100 Darmstadt (DE)
Erfinder: Würzburg, Uwe, Dr.
Dessauer Strasse 12
D-6110 Dieburg (DE)

(56) Entgegenhaltungen (fortzetzung):
CLINICAL CHEMISTRY, Band 28, Nr. 4, April 1982, Seiten 704-706, Washington, US; J. FENTON et al.: "A new chromogenic amylase method compared with two established methods"

CLINICAL CHEMISTRY, Band 28, Nr. 8, August 1982, Seiten 1787-1791, Washington, US; R. McCROSKEY et al.: "p-Nitrophenylglycosides as substrates for measurement of amylase in serum and urine"

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 133 329 B1

LIBERGRAF, STOCKHOLM 1990

**Beschreibung**

Die Erfindung betrifft ein Verfahren und ein Reagenz zur Bestimmung von α-Amylase durch enzymatische Spaltung eines substituierten nitrophenylierten Maltoheptaosids, bei dem das Aglykon β-glykosidisch gebunden ist, in Gegenwart von α-Glucosidase und β-Glucosidase und Messung des freigesetzten Aglykons.

Die Bestimmung der α-Amylaseaktivität in Körperflüssigkeiten wie z. B. Serum, Plasma, Harn, Duodenalsaft, Speichel, hat eine große Bedeutung in der klinisch-chemischen Diagnostik für die Erkennung von Pankreaserkrankungen.

Die herkömmlichen Methoden zur Bestimmung von α-Amylase beruhen darauf, daß Stärke durch α-Amylase abgebaut und die gebildeten Bruchstücke spektrophotometrisch bestimmt werden. Der wesentliche Nachteil dieser Methoden besteht darin, daß Stärke als Makromolekül schlecht zu charakterisieren und zu standardisieren ist.

Neuere Methoden verwenden deshalb definierte Oligosaccharide als Substrate, die in Gegenwart von α-Glucosidase bis zur Glucose abgebaut werden, die in an sich bekannter Weise bestimmt wird. Diese Reaktionen sind jedoch sehr komplex und werden durch endogene Glucose gestört. Eine wesentliche Vereinfachung wurde durch die Verwendung von 4-nitrophenylierten Oligosacchariden der Kettenlänge von 3 bis 12 Glucoseeinheiten (G3 - G12) als Substrat für die α-Amylase erreicht. Durch eine nachgeschaltete α-Glucosidase-Reaktion wird das freigesetzte p-Nitrophenol gemessen (DE-OS-2 731 421). Im US-Patent 4 145 527 sind Substrate für die α-Amylase mit bis zu 6 Glucoseeinheiten beschrieben, bei deren Herstellung immer ein Gemisch der α- und β-Isomere anfällt, das zusammen mit α- und β-Glucosidase eingesetzt wird. Aus Fresenius Z. Anal. Chem. *301*, 169 (1980) ist jedoch bekannt, daß mit den β–Isomeren im Gegensatz zu den α-Isomeren durchweg wesentlich kleinere Aktivitäten gemessen werden.

Wird wie im Stand der Technik vorzugsweise das p-Nitrophenol als Meßgröße verwendet, so treten zusätzliche Nachteile auf: der pKs-Wert von p-Nitrophenol liegt bei 7.09; das bedeutet, daß unter optimalen pH-Bedingungen für den Ablauf der α-Amylasebestimmung (6,8 - 7,1)' nur ca. 50 % des freigesetzten p-Nitrophenols als farbiges Phenolat-Anion vorliegen und das resultierende Meßsignal entsprechend niedrig ist. Außerdem können Änderungen in der Wasserstoffionenkonzentration des Testsystems, etwa durch Einsatz saurer oder alkalischer Proben, wie Harn oder Duodenalsaft, den Dissoziationsgrad des freigesetzten p-Nitrophenols und damit den scheinbaren molaren Extinktionskoeffizienten ändern.

Der Erfindung liegt die Aufgabe zugrunde, ein Verfahren und ein Reagenz zur Bestimmung der α-Amylase zur Verfügung zu stellen, mit dem die genannten Nachteile vermieden werden können, verbunden mit einer erheblichen Steigerung der Nachweisempfindlichkeit der α-Amylase.

Überraschenderweise wurde gefunden, daß diese Aufgabe durch die Verwendung eines substituierten 4-Nitrophenyl-β, D-maltoheptaosids als Substrat gelöst werden kann. Ebenfalls überraschend war die Tatsache, daß das β-Maltoheptaosid-Derivat ein besseres Substrat für die α-Amylase ist als das entsprechende α-Isomere, zumal aus der Literatur das Gegenteil bekannt war.

Gegenstand der Erfindung ist somit ein Verfahren zur Bestimmung von α-Amylase durch enzymatische Spaltung eines α-Amylasesubstrats in Gegenwart von α-Glucosidase und β-Glucosidase und Messung eines Spaltprodukts, das dadurch gekennzeichnet ist, daß als Substrat ein im Phenylkern elektronegativ substituiertes 4-Nitrophenyl-β, D-maltoheptaosid verwendet wird.

Ferner betrifft die Erfindung ein Reagenz zur Bestimmung von α-Amylase, enthaltend im wesentlichen ein im Phenylkern elektronegativ substituiertes 4-Nitrophenyl-β, D-maltoheptaosid, eine α-Glucosidase und eine β-Glucosidase.

Durch Einführung von elektronegativen Substituenten, d.h. von Substituenten mit -I-Effekt, wie z. B. Nitrogruppen, Cyanidgruppen, Halogene, Estergruppen, vorzugsweise Halogene wie Chlor, Brom, Fluor, insbesondere Chlor, in den 4-Nitrophenolkern wird der pKs-Wert in den sauren Bereich verschoben. Die bevorzugte Substitutionsstelle ist dabei die 2-Position im Nitrophenol. Damit erreicht man zusätzlich den Vorteil, daß das bei der Reaktion mit Glucosidase freigesetzte substituierte 4-Nitrophenol unter sonst gleichen Bedingungen eine Steigerung der Empfindlichkeit der Indikatorreaktion aufweist.

Das erfindungsgemäß bevorzugte Substrat ist das 2-Chlor-4-nitrophenyl-β, D-maltoheptaosid. Zur Abspaltung des β-ständigen Aglykons bei der α-Amylasebestimmung ist es erforderlich, daß neben der α-Glucosidase zusätzlich auch β-Glucosidase verwendet werden muß.

Unter den Testbedingungen wäre nun mit den erfindungsgemäßen Substraten ein Meßsignal zu erwarten, welches dem Verhältnis der Extinktionskoeffizienten von Halogennitrophenol und 4-Nitrophenol bei 405 nm entsprechend, um einen gewissen Faktor größer ist. Überraschenderweise ist das Meßsignal jedoch wesentlich stärker erhöht, was die Empfindlichkeit der erfindungsgemäßen α-Amylasebestimmung zusätzlich erheblich steigert. Die Abb. 1 zeigt einen Vergleich der Zeit-Umsatzkurven bei der α-Amylasebestimmung zwischen 2-Chlor-4-nitrophenyl-β, D-maltoheptaosid (I) und der entsprechenden α-Form (II) sowie mit 4-Nitrophenyl-β, D-maltoheptaosid (III). Dabei wird deutlich, daß z. B. mit 4-Nitrophenyl-β, D-maltoheptaosid eine Extinktionsänderung pro Minute von 0,024 erreicht wird; durch Chlorsubstitution im Phenylkern wird dieser Wert auf 0,036 gesteigert; der Übergang auf die β-Form läßt die Extinktionsänderung pro Minute auf über 0,075 steigen, d.h. um den Faktor 3. Neben der deutlichen Absorptionszunahme mit dem erfindungsgemäßen Substrat ist im Falle von 2-Chlor-4-nitrophenyl-β, D-maltoheptaosid ein nichtlinearer Verlauf bei der Kurve zu erkennen.

Die beiden Isoenzyme Pankreas-α-Amylase und Speichel-α-Amylase, weisen gegenüber makromolekularen Substraten unterschiedliche Aktivitäten auf. Überraschenderweise liefern diese Isoenzyme mit den erfindungsgemäßen Substraten die gleichen Aktivitäten.

Bei der α-Amylasebestimmung werden die Substrate durch die α-Amylase in kleinere Einheiten (G3, G4) gespalten, die ihrerseits durch die α-Glucosidase bis zum β-Glucosid abgebaut werden. Die Einwirkung der α-Glucosidase führt letztlich zur Abspaltung der substituierten Nitrophenolgruppe, die als Meßgröße dient.

Die Konzentration der erfindungsgemäßen Substrate im Test sollte 0,1 bis 300 mmol/l, vorzugsweise etwa 2 bis 10 mmol/l betragen. Die Konzentration der α-Glucosidase liegt üblicherweise in einem Bereich von $10^2$ bis $2{,}5 \times 10^6$ U/l, vorzugsweise bei etwa $8 \times 10^4$ U/l, die der β-Glucosidase in einem Bereich von $10^2$ bis $10^5$ U/l, vorzugsweise bei etwa 2,5 x 10 U/l.

Zur Durchführung des Verfahrens sind Puffersubstanzen erforderlich, die möglichst optimale pH-Bedingungen für den Ablauf der α-Amylasereaktion aufrechterhalten können, d. h. pH-Werte von 5 bis 9, vorzugsweise von etwa 6,5 bis 7,1. Geeignete Puffer sind z. B. Phosphatpuffer, N-(2-Hydroxyethyl)-piperazin-N-2-ethansulfonsäre (HEPES-Puffer), Imidazol, Triethanolamin, Glycerinphosphat, vorzugsweise Phosphatpuffer. Die Konzentration des Puffers sollte im Bereich von 10 bis 200 mmol/l liegen, vorzugsweise bei etwa 50 mmol/l.

Außer den genannten Bestandteilen enthält das erfindungsgemäße Reagenz ein übliches Aktivierungsmittel wie Natrium- oder Kaliumchlorid.

Zur Bestimmung von α-Amylase wird die Reagenzlösung aus Substrat, α-Glucosidase, β-Glucosidase, Puffer und Aktivierungsmittel mit der Probelösung versetzt und z. B. bei 30°C die Extinktion bei 405 nm aufgenommen.

Das erfindungsgemäße Reagenz eignet sich auch in Form von damit imprägnierten saugfähigen Materialien oder eingearbeitet in Folien als Indikator zur Bestimmung von α-Amylase.

**Beispiel 1**

Bestimmung von α-Amylase

Es wird eine Reagenzzusammensetzung hergestellt, die pro ml Testvolumen folgende Bestandteile enthält:

| Reagenz | Konzentration im Test |
|---|---|
| 2-Chlor-4-nitrophenyl-β, D-maltoheptaosid | 5 mMol/l |
| α-Glucosidase | 80 U/ml |
| β-Glucosidase | 10 U/ml |
| Phosphatpuffer (pH 6,8) | 0,05 Mol/l |
| Natriumchlorid | 0,05 Mol/l |

| | |
|---|---|
| **Meßbedingungen:** | |
| **Temperatur:** | 30°C |
| **Wellenlänge:** | 405 nm |
| **Testdurchführung:** | 1 ml Reagenz + 10 µl Probe |

Eine Zeit-Umsatzkurve mit diesem Reagenz im Vergleich zu derjenigen einer Version ohne Substitution mit Halogen im Phenylkern ist in Abb. 1 dargestellt.

Im Vergleich zur Geraden III (Substrat: 4-Nitrophenyl-α, D-maltoheptaosid) zeigt die Gerade I (Substrat: 2-Chlor-4-nitrophenyl-β, D-maltoheptaosid) eine deutliche Zunahme der Absorptionsänderung (Δ E/min ~ 0,05).

**Beispiel 2**

Bestimmung von α-Amylase, pH-Abhängigkeit der Absorption

Unter den gleichen Bedingungen wie in Beispiel 1 werden die Extinktionen von zwei auf einen pH-Wert von 7,1 eingestellten Reagenzlösungen gemessen, von denen die eine 2-Chlor-4-nitrophenol, die andere 4-Nitrophenol enthält. Anschließend werden die pH-Werte stufenweise geringfügig erhöht und erniedrigt und die Extinktionsabweichungen bestimmt. Das Ergebnis zeigt Abb. 2.

Die geringere Steigung der oberen Geraden (Reagenzlösung mit 2-Chlor-4-nitrophenol) zeigt, daß die Extinktion durch Änderung des pH-Wertes weniger stark beeinflußt wird als in der Version mit 4-Nitrophenol (untere Gerade).

**Beispiel 3**

Bestimmung von α-Amylase, Vergleich des reinen Substrats (2-Chlor-4-nitrophenyl-β-D-maltoheptaosid) mit einem Gemisch aus α- und β-Form.

Es wird die gleiche Reagenzzusammensetzung wie in Beispiel 1 hergestellt, mit dem Unterschied, daß das Substrat zu gleichen Teilen aus der α- und β-Form besteht. Der Betrag der Extinktionsänderung liegt bei Verwendung dieses Gemisches genau zwischen den Werten für die reinen Isomeren.

## Patentansprüche

1. Verfahren zur Bestimmung von α-Amylase durch enzymatische Spaltung eines α-Amylasesubstrats in Gegenwart von α-Glucosidase und β-Glucosidase und Messung eines Spaltprodukts, dadurch gekennzeichnet, daß als Substrat ein im Phenylkern elektronegativ substituiertes 4-Nitrophenyl-β, D-maltoheptaosid verwendet wird.

2. Verfahren nach Anspruch 1, dadurch gekennzeichnet, daß als Substrat ein mit Halogen substituiertes 4-Nitrophenyl-β, D-maltoheptaosid verwendet wird.

3. Verfahren nach den Ansprüchen 1 und 2, dadurch gekennzeichnet, daß als Substrat 2-Chlor-4nitrophenyl-β, D-maltoheptaosid verwendet wird.

4. Reagenz zur Bestimmung von α-Amylase, enthaltend im wesentlichen ein im Phenylkern elektronegativ substituiertes 4-Nitrophenyl-β, D-maltoheptaosid, eine α-Glucosidase und eine β-Glucosidase.

## Claims

1. Method for the determination of α-amylase by enzymatic cleavage of an α-amylase substrate in the presence of α-glucosidase and β-glucosidase and measurement of a cleavage product characterized in that a 4-nitrophenyl-β, D-maltoheptaoside electronegatively substituted in the phenyl ring is employed as the substrate.

2. Method according to Claim 1, characterised in that a 4-nitrophenyl-β, D-maltoheptaoside substituted with halogen is employed as the substrate.

3. Method according to Claims 1 and 2, characterised in that 2-chloro-4-nitrophenyl-β, D-maltoheptaoside is employed as the substrate.

4. Reagent for the determination of α-amylase, essentially containing a 4-nitrophenyl-β, D-maltoheptaoside electronegatively substituted in the phenyl ring, an α-glucosidase and a β–glucosidase.

## Revendications

1. Procédé de détermination de l'α-amylase par clivage enzymatique d'un substrat d'α-amylase en présence d'π-glucosidase et de ε-glucosidase et mesure d'un produit de clivage, caractérisé en ce qu'on utilise comme substrat un 4-nitrophényl-β, D-maltoheptoside électronégativement substitué dans le noyau phényle.

2. Procédé selon la revendication 1, caractérisé en ce qu'on utilise comme substrat un 4nitrophényl-β, D-maltoheptoside substitué par un halogène.

3. Procédé selon les revendications 1 et 2, caractérisé en ce qu'on utilise comme substrat le 2-chloro-4-nitrophényl-β, D-maltoheptoside.

4. Réactif pour la détermination de l'α-amylase, contenant pour l'essentiel un 4-nitrophényl-β, D-maltoheptoside électronégativement substitué dans le noyau phényle, une α-glucosidase et une β-glucosidase.

FIG. 1

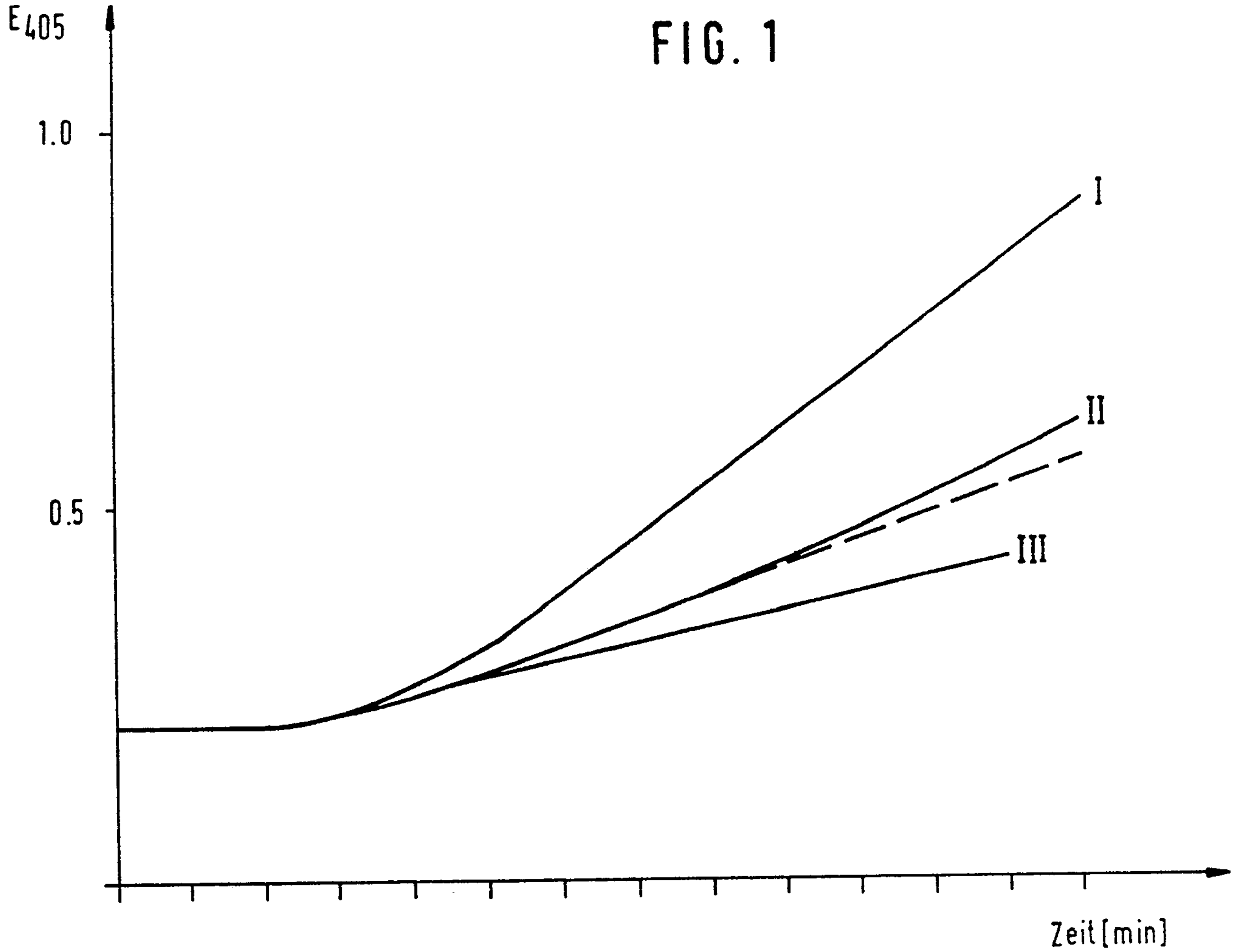


I = 2-Chlor-4-nitrophenyl-β, D-maltoheptaosid als Substrat
II = 2-Chlor-4-nitrophenyl-α, D-maltoheptaosid als Substrat
III = 4-Nitrophenyl-α, D-maltoheptaosid als Substrat

FIG. 2

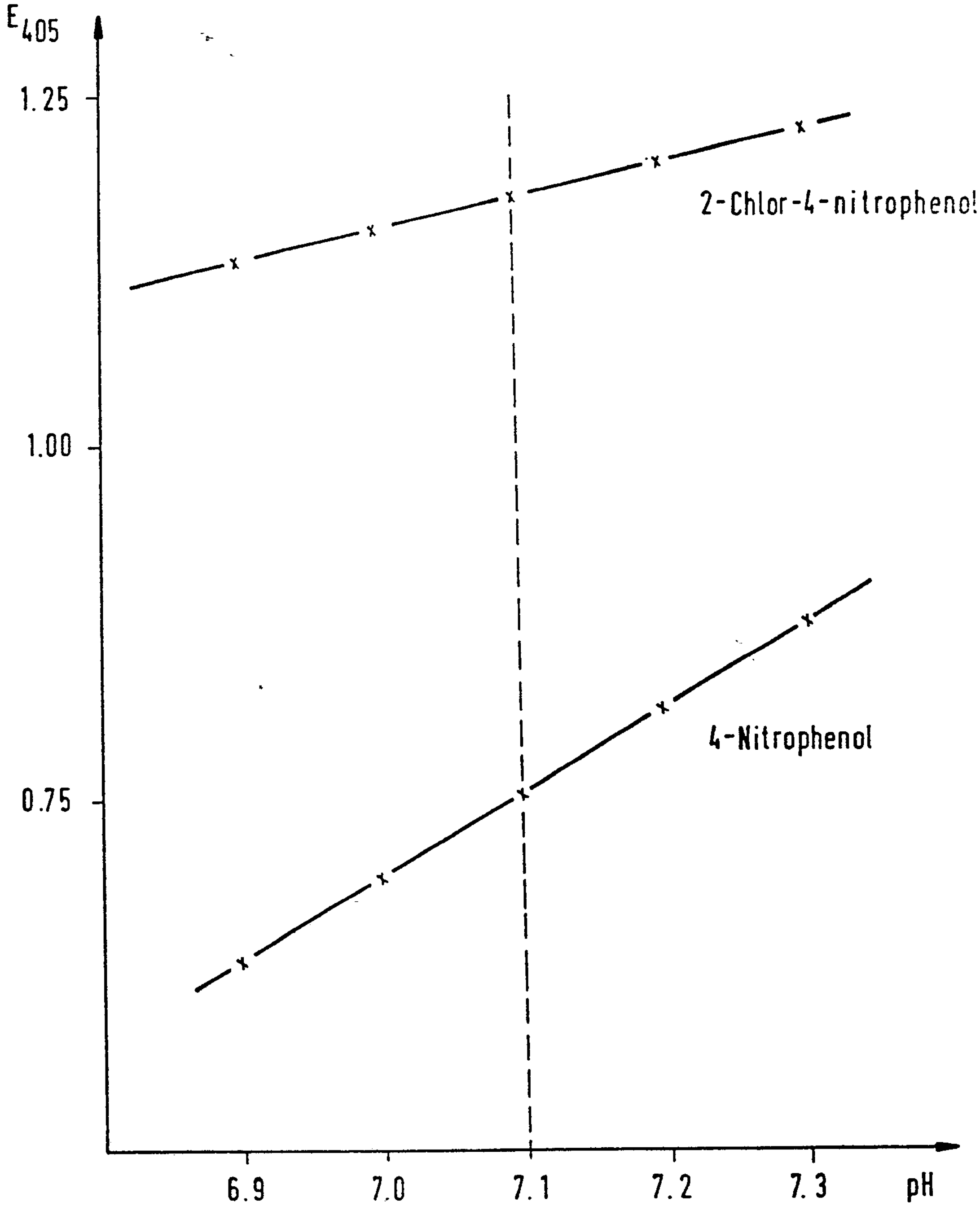
$E_{405}$
1.25
1.00
0.75
2-Chlor-4-nitrophenol
4-Nitrophenol
6.9
7.0
7.1
7.2
7.3
pH